(19) Europäisches Patentamt
European Patent Office
Office européen des brevets

(11) EP 2 014 672 A1

(12) **EUROPEAN PATENT APPLICATION**

(43) Date of publication:
**14.01.2009 Bulletin 2009/03**

(21) Application number: **07075600.2**

(22) Date of filing: **12.07.2007**

(51) Int Cl.:
***C07J 1/00*** *(2006.01)* ***A61K 31/565*** *(2006.01)*
***A61P 5/30*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Bayer Schering Pharma Aktiengesellschaft**
**13353 Berlin (DE)**

(72) Inventor: **The designation of the inventor has not yet been filed**

(54) **8-beta-substituted estratrienes as selectively active estrogens**

(57) The invention refers to 8β-substituted estra-1,3,5(10)-triene derivatives of general formula I

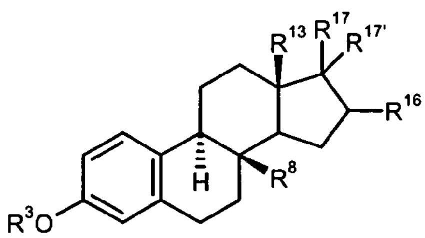

(I),

their use as pharmaceutical active ingredients, which have in vitro a higher affinity to estrogen receptor preparations from rat prostates than to estrogen receptor preparations from rat uteri and in vivo a preferential action in the ovary in comparison to the uterus, their production, their therapeutic use and pharmaceutical dispensing forms that contain the new compounds.

EP 2 014 672 A1

## Description

### Field of the Invention

This invention relates to new 8β-substituted estra-1,3,5(10)-triene derivatives of general formula

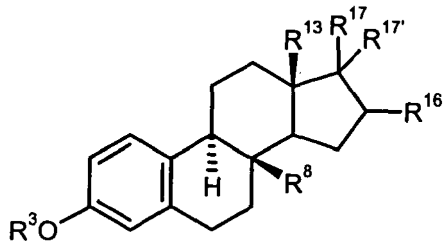

(I)

in which radicals $R^3$, $R^8$, $R^{13}$, $R^{16}$ as well as $R^{17}$ and $R^{17'}$, independently of one another, have the following meaning:

$R^3$ means a hydrogen atom or a group $R^{18}$, in which

$R^{18}$ means a straight-chain or branched-chain, saturated or unsaturated $C_1$-$C_6$-alkyl radical, a trifluoromethyl group,
an aryl, heteroaryl or aralkyl radical optionally substituted with at least one radical independently chosen from a methyl, ethyl, trifluoromethyl, pentafluoroethyl, trifluoromethylthio, methoxy, ethoxy, nitro, cyano, halo-, hydroxy, amino, mono($C_1$-$C_8$-alkyl) or di($C_1$-$C_8$-alkyl)amino whereby both alkyl groups are identical or different, di(aralkyl)amino whereby both aralkyl groups are identical or different, carboxyl, carboxyalkoxy, $C_1$-$C_{20}$-acyl or $C_1$-$C_{20}$-acyloxy as substituents;
an acyl radical -C(=O)$R^{19}$, in which $R^{19}$ is a straight-chain or branched-chain of a $C_1$-$C_{10}$ -alkyl radical that is saturated or unsaturated in up to three places and is partially or completely halogenated, or
$R^{18}$ means a group $R^{20}SO_2$, in which

$R^{20}$ is an $R^{21}R^{22}N$ group, whereby $R^{21}$ and $R^{22}$, independently of one another, mean a hydrogen atom, a $C_1$-$C_5$-alkyl radical, a group -C(=O)$R^{23}$, in which $R^{23}$ means a unsubstituted or substituted, straight-chain or branched-chain $C_1$-$C_{10}$ -alkyl radical with that is saturated or unsaturated in up to three places and is partially or completely halogenated, a cyclopropyl, cyclobutyl, cyclopentyl, cyclohexyl or cycloheptyl group, a $C_4$-$C_5$-cycloalkylalkyl radical with 3 to 7 carbon atoms in the cycloalkyl portion and with an alkyl portion of up to 8 carbon atoms or an aryl, heteroaryl or aralkyl radical, optionally substituted, with at least one radical independently chosen from a methyl, ethyl, trifluoromethyl, pentafluroethyl, trifluoromethylthio, methoxy, ethoxy, nitro, cyano, halo-, hydroxy, amino, mono($C_1$-$C_8$-alkyl) or di($C_1$-$C_8$-alkyl) amino, whereby both alkyl groups are identical or different, di(aralkyl)amino, whereby both aralkyl groups are identical or different, carboxyl, carboxyalkoxy, $C_1$-$C_{20}$-acyl or $C_1$-$C_{20}$-acyloxy as substituents; or, together with the N atom, a polymethylenimino radical with 4 to 6 carbon atoms or a morpholino radical,

$R^8$ is a straight-chain or branched-chain alkenyl or alkinyl radical with 2 to 6 carbon atoms, which optionally can be partially or completely fluorinated,
$R^{13}$ is a methyl group or an ethyl group,
$R^{16}$ is a fluorine atom,
$R^{17}$ and $R^{17'}$, in each case independently of one another, are a hydrogen atom and a hydroxy group; or
a hydrogen atom and a group $R^{18}O$-, $R^{20}SO_2$- or OC(O)$R^{23}$ with $R^{18}$, $R^{20}$ and $R^{23}$ in each case in the meaning that is indicated under $R^3$.

This invention further relates to the use of the new 8β-substituted estra-1,3,5(10)-triene as pharmaceutical active ingredients, which have in vitro a higher affinity to estrogen receptor preparations from rat prostates than to estrogen receptor preparations from rat uteri and in vivo a preferential action in the ovary in comparison to the uterus, their production, their therapeutic use and pharmaceutical dispensing forms that contain the new compounds.

The 8β-substituted estra-1,3,5(10)-triene derivatives of the present inventionare new, steroidal, estrogen receptor subtype -selective estratriene with improved potency and metabolic stability.

**Background of the Invention**

The efficiency of estrogens in the treatment of hormone-deficiency-induced symptoms such as hot flashes, atrophy of estrogen target organs and incontinence, as well as the successful use of estrogen therapies for prevention of bone mass loss in peri- and postmenopausal women, is well documented and generally accepted (Grady et al. 1992, Ann Intern Med 117: 1016-1037). It is also well documented that estrogen replacement therapy in postmenopausal women or in women with ovarian dysfunction that is caused in some other way reduces the risk of cardiovascular diseases compared to women who are not treated with estrogen (Grady et al., loc. cit.).

In conventional estrogen or hormone replacement therapy (= HRT), natural estrogens, such as estradiol, and conjugated estrogens that consist of equine urine are used either by themselves or in combination with a gestagen. Instead of the natural estrogens, derivatives that are obtained by esterification, such as, e.g., 17β-estradiol-valerate, can also be used.

Because of the stimulating action of the estrogens that are used on the endometrium, which results in an increase of the risk of endometrial carcinoma (Harlap, S. 1992, Am J Obstet Gynecol 166: 1986-1992), estrogen/gestagen combination preparations are preferably used in hormone replacement therapy. The gestagenic component in the estrogen/gestagen combination avoids hypertrophy of the endometrium, but the occurrence of undesirable intracyclic menstrual bleeding is also linked to the gestagen-containing combination.

Selective estrogens represent a more recent alternative to the estrogen/gestagen combination preparations. Up until now, selective estrogens have been defined as those compounds that have an estrogen-like effect on the brain, bones and vascular system, owing to their antiuterotropic (i.e., antiestrogenic) partial action, but they do not have a proliferative effect on the endometrium.

A class of substances that partially meet the desired profile of a selective estrogen is the so-called "Selective Estrogen Receptor Modulators" (SERM) (R. F. Kauffman, H. U. Bryant 1995, DNAP 8 (9): 531-539). In this case, these are partial agonists of estrogen receptor subtype "ERα." This substance type is ineffective, however, with respect to the therapy of acute postmenopausal symptoms, such as, e.g., hot flashes. As an example of a SERM, the raloxifene that was recently introduced for the indication of osteoporosis can be mentioned.

For the treatment of fertility disorders of women, frequently caused by ovarian dysfunction that is caused by surgery, medication, etc., new possible therapies are also opened up by the use of new selective estrogens. The in-vitro fertility treatment is a process that has been established for more than 20 years. Numerous methods for treating ovarian-induced infertility with exogenic gonadotropins are known. By administration of gonadotropins such as FSH (FSH = follicle-stimulating hormone), a stimulation of the ovaries, which is to make possible a healthy follicular maturation, is to be produced.

The follicle is the functional unit of the ovary and has two purposes: it accommodates the oocytes and provides for the latter the possibility for growth and for maturation. Folliculogenesis comprises the development of an ovarian follicle from a primordial stage to a continuously increasing antral follicle, which represents the last stage before ovulation. Only an optimally developed antral follicle can release a mature oocyte by ovulation.

Patients with ovarian-induced infertility (PCOS = syndrome of polycystic ovaries) suffer from a disrupted follicular maturation, which is associated both with hormonal and ovulatory disruptions and with inadequately matured oocytes. The number of primary and secondary follicles is approximately twice as high here as in the normal ovary (Hughesden et al., Obstet. Gynecol. Survey 37, 1982, pp. 59-77).

There are indications that the early development stages of folliculogenesis (which relates to the development of primordial follicles to antral follicles) are gonadotropin-independent. It is not clearly explained how great the influence of known paracrine and autocrine factors is on early folliculogenesis (Elvin et al., Mol. Cell Endocrinol. 13, 1999, pp. 1035-1048; McNatty et al., J. Reprod. Fertil. Suppl. 54, 1999, pp. 3-16).

Gonadotropins such as FSH are mainly involved in the last development stages of folliculogenesis in follicular maturation, i.e., in the development of the early antral follicle to a mature follicle that can undergo ovulation.

The in-vivo and in-vitro infertility is preferably treated with gonadotropins (FSH and antiestrogens) (White et al., J. Clin. Endocrinol. Metab. 81, 1996, pp. 3821-3824). In in-vitro fertilization treatment, oocytes are removed from preovulatory antral follicles to be able to mature in vitro into an oocyte that can be fertilized. After fertilization and early embryonic development, one to three embryos are implanted in the uterus of the woman.

In many respects, the treatment with exogenic gonadotropins is accompanied by numerous risks and side effects. The greatest risk consists in an overstimulation of the ovaries, which in severe cases can represent a serious danger to life (OHSS = Ovarian Hyperstimulation Syndrome). Other side effects are the high costs of the in-vitro fertility treatment that must be paid by the couples. Negative side effects such as weight gain, bloatedness, nausea, vomiting and an as yet unknown long-term risk of developing cancer are attributed to the gonadotropin treatment.

One method to avoid the above-mentioned drawbacks and risks is to ensure the maturation and stimulation in vivo of follicular growth in the case of ovarian-induced infertility with a suitable active ingredient before treatment with exogenic gonadotropins begins.

**Estrogen Receptor Beta (ERβ)**

Several years ago, estrogen receptor β (ERβ) was discovered as a second subtype of the estrogen receptor (Kuiper et al. (1996), Proc. Natl. Acad. Sci. 93: 5925-5930; Mosselman, Dijkema (1996) Febs Letters 392: 49-53; Tremblay et al. (1997), Molecular Endocrinology 11: 353-365). The expression pattern of ERβ differs from that of the ERα (Kuiper et al. (1996), Endocrinology 138: 863-870). ERβ thus predominates over ERα in the rat prostate, while ERα predominates over ERβ in the rat uterus. The highest concentrations of ERβ and mRNA were found in the ovaries (Couse et al. Endocrinology 138, 1997, pp. 4612-4613).

Other organ systems with comparatively higher ERβ-expression comprise the bones (Onoe, Y. et al., 1997, Endocrinology 138: 4509-4512), the vascular system (Register, T. C., Adams, M. R. 1998, J. Steroid Molec Biol 64: 187-191), the urogenital tract (Kuiper, G. J. M. et al. 1997, Endocrinology 138: 863-870), the gastrointestinal tract (Campbell-Thopson 1997, BBRC 240: 478-483), as well as the testis (Mosselmann, S. et al. 1996 FEBS Lett. 392, 49-53) including the spermatides (Shugrue et al. 1998, Steroids 63: 498-504). The tissue distribution suggests that estrogens regulation of organ functions via ERβ is highly relevant. The fact that ERβ is functional in this respect also follows by studies in ERα- (ERKO) or ERβ-(βERKO)-knockout mice: ovariectomy produces bone mass loss in ERKO-mice, which can be eliminated by estrogen substitution (Kimbro et al. 1998, Abstract OR7-4, Endocrine Society Meeting, New Orleans). Estradiol in the blood vessels of female ERKO mice also inhibits vascular media and smooth muscle cell proliferation (Iafrati, M. D. et al. 1997, Nature Medicine 3: 545-548). These protective actions of estradiol are carried out in the ERKO mouse presumably via ERβ.

The fact that ERα and ERβ have a functionally different action was confirmed after successful production of ERKO and βERKO mice. ERα consequently plays an important role in the adult uterus, in mammary gland tissue, in the negative regulation of the gonadotropin activity, while ERβ is mainly bonded in the processes of ovarian physiology, especially that of folliculogenesis and ovulation (Couse et al., Endocrine Reviews 20, 1999, pp. 358-417).

Observations of βERKO mice provide an indication on a function of ERβ in the prostate and bladder: in the case of older male mice, symptoms of prostate and bladder hyperplasia occur (Krege, J. H. et al. 1998, Proc Natl Acad Sci 95: 15677-15682). In addition, female ERKO mice (Lubahn, D. B. et al. 1993, Proc Natl Acad Sci 90: 11162-11166) and male ERKO mice (Hess, R. A. et al. 1997, Nature 390: 509-512) as well as female βERKO mice (Krege, J. H., 1998, Proc Natl Acad Sci 95: 15677-15682) have fertility disorders. Consequently, the important function of estrogens with respect to maintaining testis and ovary functions as well as fertility is confirmed.

It is possible to achieve a selective estrogenic action on specific target organs by subtype-specific ligands based on the different tissue or organ distribution of the two subtypes of the ERs. Substances with a preference for ERβ compared to ERα in the in-vitro receptor binding test were described by Kuiper et al. (Kuiper et al. (1996), Endocrinology 138: 863-870). A selective action of subtype-specific ligands of the estrogen receptor on estrogen-sensitive parameters in vivo was not previously shown.

The patent application WO 01/77139 A1 describes 8β-substituted estratrienes wherein $R^8$ means a straight-chain or branched-chain, optionally partially or completely halogenated alkyl or alkenyl radical with up to 5 carbon atoms, an ethinyl- or prop-1-inyl radical, as pharmaceutical active ingredients that have in vitro a higher affinity to estrogen receptor preparations of rat prostates than to estrogen receptor preparations of rat uteri, their production, their therapeutic use and pharmaceutical dispensing forms that contain the said compounds. Compounds with potent estrogen activity and a higher affinity to estrogen receptor preparations of rat prostates than to estrogen receptor preparations of rat uteri for the production of medicaments are highly needed in the present technical field.

**Object of the Invention**

The object of this invention is therefore to prepare compounds that have in vitro a high dissociation with respect to the binding to estrogen receptor preparations from rat prostates and rat uteri. The compounds are to show in vitro a higher affinity to estrogen receptor preparations from rat prostates than to estrogen receptor preparations from rat uteri. The compounds according to the present invention have a high potent estrogen activity and efficacy, namely
a higher binding affinity to rat prostate estrogen receptor and a better dissociation regarding binding to rat prostate versus rat uterus estrogen receptor with respect to the known compounds. The compounds according to the invention are to produce enhanced fertility in the ovary while at the same time affecting the uterus very little in cases of ovarian-associated infertility.

The advantageous profile of the compounds according to the invention was achieved by the specific combination of the substituents $R^8$, $R^{13}$, $R^{16}$, $R^{17}$ and $R^{17'}$.

According to the invention, the object above is achieved by the provision of 8β-substituted estra-1,3,5(10)-triene derivatives of general formula I

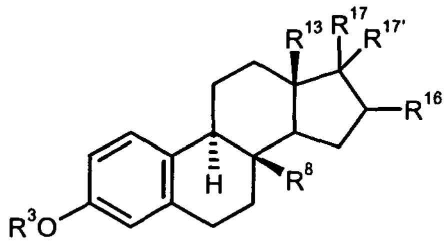

(I)

in which radicals $R^3$, $R^8$, $R^{13}$, $R^{16}$ as well as $R^{17}$ and $R^{17'}$, independently of one another, have the following meaning:

$R^3$ means a hydrogen atom or a group $R^{18}$, in which

$R^{18}$ means a straight-chain or branched-chain, saturated or unsaturated $C_1$-$C_6$-alkyl radical, a trifluoromethyl group, an aryl, heteroaryl or aralkyl radical optionally substituted with at least one radical independently chosen from a methyl, ethyl, trifluoromethyl, pentafluoroethyl, trifluoromethylthio, methoxy, ethoxy, nitro, cyano, halo-, hydroxy, amino, mono($C_1$-$C_8$-alkyl) or di($C_1$-$C_8$-alkyl)amino whereby both alkyl groups are identical or different, di(aralkyl)amino whereby both aralkyl groups are identical or different, carboxyl, carboxyalkoxy, $C_1$-$C_{20}$-acyl or $C_1$-$C_{20}$-acyloxy as substituents; an acyl radical -C(=O)$R^{19}$, in which $R^{19}$ is a straight-chain or branched-chain of a $C_1$-$C_{10}$ -alkyl radical that is saturated or unsaturated in up to three places and is partially or completely halogenated, or
$R^{18}$ means a group $R^{20}SO_2$, in which

$R^{20}$ is an $R^{21}R^{22}N$ group, whereby $R^{21}$ and $R^{22}$, independently of one another, mean a hydrogen atom, a $C_1$-$C_5$ -alkyl radical, a group -C(=O)$R^{23}$, in which $R^{23}$ means a unsubstituted or substituted, straight-chain or branched-chain $C_1$-$C_{10}$ -alkyl radical that is saturated or unsaturated in up to three places and is partially or completely halogenated, a cyclopropyl, cyclobutyl, cyclopentyl, cyclohexyl or cycloheptyl group, a $C_4$-$C_{15}$-cycloalkylalkyl radical with 3 to 7 carbon atoms in the cycloalkyl portion and with an alkyl portion of up to 8 carbon atoms or an aryl, heteroaryl or aralkyl radical, optionally substituted with at least one radical independently chosen from a methyl, ethyl, trifluoromethyl, pentafluroethyl, trifluoromethylthio, methoxy, ethoxy, nitro, cyano, halo- , hydroxy, amino, mono($C_1$-$C_8$-alkyl) or di($C_1$-$C_8$-alkyl) amino, whereby both alkyl groups are identical or different, di(aralkyl)amino, whereby both aralkyl groups are identical or different, carboxyl, carboxyalkoxy, $C_1$-$C_{20}$-acyl or $C_1$ -$C_{20}$-acyloxy groups as substituents; or, together with the N atom, a polymethylenimino radical with 4 to 6 carbon atoms or a morpholino radical,

$R^8$ is a straight-chain or branched-chain alkenyl or alkinyl radical with 2 to 6 carbon atoms, which optionally can be partially or completely fluorinated,
$R^{13}$ is a methyl group or an ethyl group,
$R^{16}$ is a fluorine atom,
$R^{17}$ and $R^{17'}$, in each case independently of one another, are a hydrogen atom and a hydroxy group; or a hydrogen atom and a group $R^{18}O$-, $R^{20}SO_2$- or OC(O)$R^{23}$ with $R^{18}$, $R^{20}$ and $R^{23}$ in each case in the meaning that is indicated under $R^3$.

A particular embodiment of the present invention are the compounds of general formula I, in which $R^3$ is a hydrogen atom.

According to a further embodiment of the invention, compounds of general formula I comprise $R^8$ being a vinyl, ethinyl or prop-1-inyl group.

Other possible forms of embodiment of the present invention are defined by the depending claims.

Compounds of general formula I in which $R^{17}$ and $R^{17'}$ are a hydrogen atom and a hydroxy group atom are further preferred.

Compounds of general formula I in which $R^{16}$ is in α-position or compounds of general formula I in which $R^{16}$ is in β-position are equally preferred forms of embodiment of the invention.

Furthermore, a particular embodiments of the present invention are the compounds of general formula I in which $R^8$ is a vinyl, ethinyl or prop-1-inyl group, $R^{16}$ is a fluorine atom, $R^{17}$ and $R^{17'}$ are independently from one another a hydrogen atom and a hydroxy group atom.

Compounds of general formula I in which $R^{17}$ and/or $R^{17'}$ are a hydrogen atom and a group $R^{18}$-O- or $R^{19}SO_2$-O- with $R^{18}$ and $R^{19}$ in each case in the meaning that is indicated under $R^3$ are further particular form of embodiment of the present invention.

Another variant of the invention in particular calls for compounds in which $R^{17}$ stands for a group $R^{18}$O- or $R^{20}SO_2$-O- with $R^{18}$ and $R^{20}$ in each case in the meaning that is indicated under $R^3$.

Compounds according to the present invention are:

8β-vinyl-16α-fluoro-estra-1,3,5(10)-triene-3,17α-diol
8β-vinyl-16α-fluoro-estra-1,3,5(10)-triene-3,17β-diol
8β-vinyl-16β-fluoro-estra-1,3,5(10)-triene-3,17β-diol

As halo- or halogen, a fluorine, chlorine, bromine or iodine atom is intended according to the present invention

As alkyl radical is generally intended a $(C_1-C_6)$alkyl radical, as far as it is not differently specified, said alkyl radical being a straight or branched chain, saturated or unsaturated. Representative groups for an alkyl radical according to the present invention are methyl, ethyl, n- propyl, 1-methylethyl (iso-propyl), n-butyl, n-pentyl, 1,1-dimethylethyl (t-butyl) and n-hexyl. The $C_1$-$C_6$-alkyl radical can be partially or completely substituted by halogen atoms, hydroxy groups or $C_1$-$C_6$-alkoxy groups.

According to the above definition $R^{18}$ is, for example, a methyl, ethyl, propyl, isopropyl, butyl, isobutyl, tert.-butyl, pentyl, isopentyl, neopentyl, or hexyl radical.

Alkoxy groups $OR^{18}$ in the compounds of general formula I in each case can contain an alkyl radical according to the definition given above, whereby methoxy, ethoxy, propoxy, isopropoxy and t-butyloxy groups are preferred alkoxy radical.

Representatives of the $C_1$-$C_5$-alkyl radicals $R^{21}$ and $R^{22}$ are methyl, ethyl, propyl, isopropyl, butyl, isobutyl, tert-butyl, pentyl, isopentyl and neopentyl.

As representatives of straight-chain or branched-chain $C_1$-$C_{10}$ -alkyl radicals $R^{23}$, for example, methyl, ethyl, propyl, isopropyl, butyl, isobutyl, tert-butyl, pentyl, isopentyl, neopentyl, heptyl, hexyl, and decyl can be mentioned; methyl, ethyl, propyl and isopropyl are preferred.

As a $C_3$-$C_7$-cycloalkyl group, a cyclopropyl, cyclobutyl, cyclopentyl, cyclohexyl or cycloheptyl group can be mentioned.

A $C_4$-$C_{15}$-cycloalkylalkyl radical has 3 to 7 carbon atoms in the cycloalkyl portion; typical representatives are the cycloalkyl groups that are mentioned directly above. The alkyl portion has up to 8 carbon atoms.

As examples of a $C_4$-$C_{15}$-cycloalkylalkyl radical, the cyclopropylmethyl, cyclopropylethyl, cyclopentylmethyl, cyclopentylpropyl groups, etc., can be mentioned.

In terms of this invention, an aryl radical is a phenyl, 1- or 2-naphthyl radical; the phenyl radical is preferred.

Examples of a heteroaryl radical according to the present invention are the 2-, 3- or 4-pyridinyl, the 2- or 3-furyl, the 2- or 3-thienyl, the 2-or 3-pyrrolyl, the 2-, 4- or 5-imidazolyl, the pyrazinyl, the 2-, 4- or 5-pyrimidinyl or 3- or 4-pyridazinyl radical.

As substituents that can be present on an aryl or heteroaryl radical, for example, a methyl-, ethyl-, trifluoromethyl-, pentafluoroethyl-, trifluoromethylthio-, methoxy-, ethoxy-, nitro-, cyano-, halogen- (fluorine, chlorine, bromine, iodine), hydroxy-, amino-, mono($C_1$-$C_8$-alkyl) or di($C_1$-$C_8$-alkyl)amino, whereby both alkyl groups are identical or different, di(aralkyl)amino, whereby both aralkyl groups are identical or different, carboxyl, carboxyalkoxy, $C_1$-$C_{20}$-acyl or $C_1$-$C_{20}$-acyloxy groups can be mentioned.

An aralkyl radical is a radical that contains in the ring up to 14, preferably 6 to 10 C atoms, and in the alkyl chain 1 to 8, preferably 1 to 4, C atoms. Thus, as aralkyl radicals, for example, benzyl, phenylethyl, naphthylmethyl, naphthylethyl, furylmethyl, thienylethyl, and pyridylpropyl are suitable.

The alkyl groups and radicals can be partially or completely substituted by 1-5 halogen atoms, hydroxy groups or $C_1$-$C_4$-alkoxy groups.

A vinyl or allyl radical is primarily defined with a $C_2$-$C_6$-alkenyl radical.

A $C_2$-$C_6$-alkinyl radical is preferably defined as an ethinyl radical or a prop-1-inyl radical.

$C_{1-10}$-Acyl radicals mean, for example, acetyl, propionyl, butyryl, valeroyl, isovaleroyl, pivaloyl, hexanoyl, octyl, nonyl, or decanoyl.

One or two hydroxyl groups at C atoms 3 and 16 can be esterified with an aliphatic, straight-chain or branched-chain, saturated or unsaturated $C_1$-$C_{14}$-mono- or polycarboxylic acid or an aromatic carboxylic acid.

Suitable as such carboxylic acids for esterification are, for example:

- Monocarboxylic acids: formic acid, acetic acid, propionic acid, butyric acid, isobutyric acid, valeric acid, isovaleric acid, pivalic acid, lauric acid, myristic acid, acrylic acid, propionic acid, methacrylic acid, crotonic acid, isocrotonic

acid, oleic acid, and elaidic acid. Esterification with acetic acid, valeric acid or pivalic acid is preferred.

- Dicarboxylic acids: oxalic acid, malonic acid, succinic acid, glutaric acid, adipic acid, pimelic acid, suberic acid, azelaic acid, sebacic acid, maleic acid, fumaric acid, muconic acid, citraconic acid, and mesaconic acid.
- Aromatic carboxylic acids: benzoic acid, phthalic acid, isophthalic acid, terephthalic acid, naphthoic acid, o-, m- and p-toluic acid, hydratropic acid, atropic acid, cinnamic acid, nicotinic acid, and isonicotinic acid. Esterification with benzoic acid is preferred.

As prodrugs, the esters of the 8β-substituted estratrienes according to the invention have advantages compared to the unesterified active ingredients with respect to their method of administration, their type of action, strength and duration of action.

Especially the sulfamates of 8β-substituted estratrienes according to the invention have pharmacokinetic and pharmacodynamic advantages. Related effects were already described in other steroid-sulfamates (J. Steroid Biochem. Molec. Biol, 55, 395-403 (1995); Exp. Opinion Invest. Drugs 7, 575-589 (1998)).

In this patent application, steroids based on a 8β-substituted estra-1,3,5(10)-triene skeleton are described for the treatment of estrogen receptor β-mediated disorders and conditions as selective estrogens, which have in-vitro dissociation with respect to their binding to estrogen receptor preparations from rat prostates and rat uteri and which have in vivo preferably a dissociation with respect to ovary action in comparison to uterus action.

The ERβ-specific compounds according to the present invention are to mediate in vivo a profertility action in the ovary. At the same time, the compounds are to exhibit a dissociation with respect to ovary action in comparison to uterus action.

It was found that the 8β-substituted estra-1,3,5(10)-trienes according to general formula I are suitable as selective estrogens for the treatment of various conditions and disorders that are characterized by a higher content of estrogen receptor β than estrogen receptor α in the corresponding target tissue or target organ. Said compounds have improved potency and metabolic stability.

The invention also relates to pharmaceutical preparations that contain at least one compound of general formula I (or physiologically compatible addition salts with organic or inorganic acids thereof) and the use of the compounds of general formula I for the production of pharmaceutical agents, especially for the indications mentioned below.

The new selective estrogens that are described here can be used as individual components in pharmaceutical preparations or in combination especially with gestagens. Part of the present invention is the combination of selective estrogens with ERα-selective antiestrogens that are peripherally-selectively active, i.e., that do not pass through the blood-brain barriers, as well as with selective estrogen receptor modulators (SERM).

The ERβ-selective compounds according to the invention can be used in particular for the production of pharmaceutical agents for treating fertility disorders, for prevention and therapy of prostate hyperplasia, for prevention and treatment of hormone-deficiency-induced mood swings in women and men and for use in hormone replacement therapy (HRT) in men and women.

Furthermore, the compounds according to the invention have antiproliferative effects in models of colon and intestinal hyperplasia and can therefore be administered for the prevention and treatment of diseases associated with colon and intestinal epithelium proliferation like for the treatment and the prevention of cancer.

The ERβ-specific compounds according to the present invention can be advantageously used for the selective stimulation of hair growth.

A therapeutic product that contains an estrogen and a pure antiestrogen for simultaneous, sequential or separate use for the selective estrogen therapy of perimenopausal or postmenopausal conditions is already described in EP-A 0 346 014.

Because of their dissociation of action in the ovary in comparison to the action of the uterus, the substances and the pharmaceutical agents that contain them are especially suitable for the treatment in the case of ovarian dysfunction that is caused by surgery, medication, etc., such as female infertility for stimulation of folliculogenesis for treatment by itself in terms of enhanced fertility, for supporting in-vitro fertility treatment (IVF) in connection with an in-vivo treatment and for treatment of ovarian-induced disorders in later age ("late fertility") as well as for treatment of hormone-deficiency-induced symptoms.

The compounds of this invention are also suitable for therapy of ovarian diseases such as polycystic ovarian syndrome, POF (premature ovarian failure) syndrome, and ovulation disorders.

Finally, the compounds of general formula I can be used in connection with selective estrogen receptor modulators (SERM) or raloxifene, specifically in particular for use in hormone replacement therapy (HRT) and for treatment of gynecological disorders.

The 8β-substituted estratrienes according to the invention are also suitable as individual components for the treatment of perimenopausal and postmenopausal symptoms, in particular hot flashes, sleep disturbances, irritability, mood swings, incontinence, vaginal atrophy and hormone-deficiency-induced mental disorders. The above 8β-substituted estratrienes are also suitable for hormone substitution and for the therapy of hormone-deficiency-induced symptoms

in ovarian dysfunction that is caused by surgery, medication, etc.

In addition, the 8β-substituted estratrienes according to the invention can also be used to prevent cardiovascular diseases, in particular vascular diseases such as arteriosclerosis, high blood pressure, hypertensive heart disease and to prevent hormone-deficiency-induced neurodegenerative diseases, such as Alzheimer's disease, as well as hormone-deficiency-induced impairment of memory and learning capacity.

In addition, the compounds according to the present invention can be used as active ingredients in preparations for treating inflammatory diseases and diseases of the immune system, in particular autoimmune diseases, such as, e.g., rheumatoid arthritis, multiple sclerosis, lupus, Crohn's disease and other inflammatory intestinal diseases, inflammatory diseases of the skin, such as psoriasis, as well as for treating endometriosis. Building on the evidence from preclinical models of human inflammatory diseases, the ERβ-specific compounds can therfore be used for the prevention and treatment of the diseases mentioned above (Heather, H.A.; Mol Endocrinol. 2007 Jan;21(1):1-13. Epub 2006 Mar 23. Review)..

In addition, the compounds are effective against inflammatory diseases of the respiratory system, the lungs and bronchial tubes, such as, e.g., asthma.

The medication is suitable for therapy and prophylaxis of estrogen-deficiency-induced diseases both in women and in men.

The present compounds are also suitable for prevention and therapy of prostate hyperplasia.

The compounds can be further used for prophylaxis and therapy of humans age-related dysfunctions or diseases. In particular, they can be used for prevention and treatment of an age-related drop of androgens, such as testosterone and DHEA, as well as of the growth hormone.

The amount of a compound of general formula I that is to be administered fluctuates within a wide range and can cover any effective amount. On the basis of the condition that is to be treated and the type of administration, the amount of the compound that is administered can be 0.01 μg/kg- 100 mg/kg of body weight, preferably 0.04 μg/kg- 1 mg/kg of body weight, per day.

In humans, this corresponds to a dose of 0.8 μg to 8 g, preferably 3.2 μg to 80 mg, daily.

According to the invention, a dosage unit contains 1.6 μg to 2000 mg of one or more compounds of general formula I.

The compounds according to the invention and the acid addition salts are suitable for the production of pharmaceutical compositions and preparations. The pharmaceutical compositions or pharmaceutical agents contain as active ingredients one or more of the compounds according to the invention or their acid addition salts, optionally mixed with other pharmacologically or pharmaceutically active substances. The production of the pharmaceutical agents is carried out in a known way, whereby the known and commonly used pharmaceutical adjuvants as well as other commonly used vehicles and diluents can be used.

As such vehicles and adjuvants, for example, those are suitable that are recommended or indicated in the following bibliographic references as adjuvants for pharmaceutics, cosmetics and related fields: Ullmans Encyklopädie der technischen Chemie [Ullman's Encyclopedia of Technical Chemistry], Volume 4 (1953), pages 1 to 39; Journal of Pharmaceutical Sciences, Volume 52 (1963), page 918 ff., issued by Czetsch-Lindenwald, Hilfsstoffe für Pharmazie und angrenzende Gebiete [Adjuvants for Pharmaceutics and Related Fields]; Pharm. Ind., Issue 2, 1961, p. 72 and ff.: Dr. H. P. Fiedler, Lexikon der Hilfsstoffe für Pharmazie, Kosmetik und angrenzende Gebiete [Dictionary of Adjuvants for Pharmaceutics, Cosmetics and Related Fields], Cantor KG, Aulendorf in Württemberg 1971.

The compounds can be administered orally or parenterally, for example intraperitoneally, intramuscularly, subcutaneously or percutaneously. The compounds can also be implanted in the tissue.

For oral administration, capsules, pills, tablets, coated tablets, etc., are suitable. In addition to the active ingredient, the dosage units can contain a pharmaceutically compatible vehicle, such as, for example, starch, sugar, sorbitol, gelatin, lubricant, silicic acid, talc, etc.

For parenteral administration, the active ingredients can be dissolved or suspended in a physiologically compatible diluent. As diluents, very often oils with or without the addition of a solubilizer, a surfactant, a suspending agent or an emulsifying agent are used. Examples of oils that are used are olive oil, peanut oil, cottonseed oil, soybean oil, castor oil and sesame oil.

The compounds can also be used in the form of a depot injection or an implant preparation, which can be formulated so that a delayed release of active ingredient is made possible.

As inert materials, implants can contain, for example, biodegradable polymers, or synthetic silicones such as, for example, silicone rubber. In addition, for percutaneous administration, the active ingredients can be added to, for example, a patch.

For the production of intravaginal systems (e.g., vaginal rings) or intrauterine systems (e.g., pessaries, coils, IUDs, Mirena$^{(R)}$) that are loaded with active compounds of general formula I for local administration, various polymers are suitable, such as, for example, silicone polymers, ethylene vinyl acetate, polyethylene or polypropylene.

To achieve better bio-availability of the active ingredient, the compounds can also be formulated as cyclodextrin

clathrates. For this purpose, the compounds are reacted with α-, β-, or γ-cyclodextrin or derivatives of the latter (PCT/EP95/02656).

According to the invention, the compounds of general formula I can also be encapsulated with liposomes.

**Methods**

**Estrogen Receptor Binding Studies:**

The binding affinity of the new selective estrogens was tested in competitive experiments with use of $^3$H-estradiol as a ligand to estrogen receptor preparations from rat prostates and rat uteri. The preparation of prostate cytosol and the estrogen receptor test with prostate cytosol was carried out as described by Testas et al. (1981) (Testas, J. et al., 1981, Endocrinology 109: 1287-1289).

The preparation of rat uterus cytosol as well as the receptor test with the ER-containing cytosol were basically performed as described by Stack and Gorski, (1985) (Stack, Gorski 1985, Endocrinology 117, 2024-2032) with some modifications as described in Fuhrmann et al. (1995) (Fuhrmann, U. et al. 1995, Contraception 51: 45-52).

The substances that are described here have higher binding affinity to the estrogen receptor of rat prostates than to estrogen receptors of rat uteri. In this case, it is assumed that ERβ predominates in the rat prostates over ERα, and ERα predominates in rat uteri over ERβ. Table 1 shows that the ratio of the binding to prostate and uterus receptors qualitatively coincides with the quotient of relative binding affinity (RBA) to human ERβ and ERα of rats (according to Kuiper et al. (1996), Endocrinology 138: 863-870) (Table 1).

Table 1

| Estrogen | Structure | hERα RBA* | hERβ RBA* | ERβ/ ERα | Rat uterus ER(RBA) | Rat prost. ER(RBA) | prost. ER/ uterus ER |
|---|---|---|---|---|---|---|---|
| **Estradiol** | 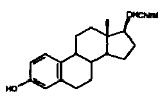 | 100 | 100 | 1 | 100 | 100 | 1 |
| **Estrone** | 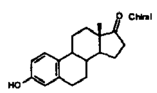 | 60 | 37 | 0.6 | 3 | 2 | 0.8 |
| **17α-Estra-diol** | 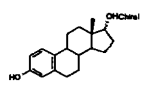 | 58 | 11 | 0.2 | 2.4 | 1.3 | 0.5 |
| **Estriol** | 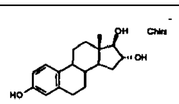 | 14 | 21 | 1.5 | 4 | 20 | 5 |
| **5-Andro-stene-diol** | 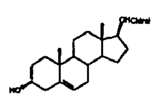 | 6 | 17 | 3 | 0.1 | 5 | 50 |
| **Genisteine** | 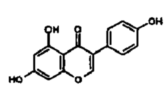 | 5 | 36 | 7 | 0.1 | 10 | 100 |
| **Coumes-trol** | 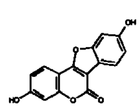 | 94 | 185 | 2 | 1.3 | 24 | 18 |

*Cited from: Kuiper et al. (1996), Endocrinology 138: 863-870

Table 2 shows the results for one of the 8β-vinyl-estra-1,3,5(10)-triene-3,17-diol derivatives (compounds 1) according to the invention. Compound 2(8β-vinyl-estra-1,3,5(10)-triene-3,17-diol is shown as a reference.

**Table 2**

| Compound | Rat uterus ER(RBA) | Rat prost. ER(RBA) | prost. ER/uterus ER |
|---|---|---|---|
| 8β-Vinyl-estra-1,3,5(10)-16α-fluoro-3,17β-diol (1) | 1.7 | 167 | 98 |
| 8β-Vinyl-estra-1,3,5(10)-3,17β-diol (2) | 0.7 | 63 | 90 |

Compounds 1 according to the invention as well as compound 2 show a higher binding affinity to the estrogen receptor of rat prostates than to the estrogen receptor of rat uteri. Compound 1 is superior to compound 2 by a higher binding affinity to rat prostate estrogen receptor and by a better dissociation regarding binding to rat prostate versus rat uterus estrogen receptor.

In addition, the predictability of the prostate-ER versus the uterus-ER test system is confirmed with respect to tissue-selective action by in-vivo studies. Substances with a preference for prostate-ER are dissociated in vivo preferably with respect to ovary and uterus action as well as pituitary gland action in favor of action on the ovary.

**Studies for Dissociation of Action of the Uterus and Pituitary Gland**

The studies with respect to the action on uterus growth and ovulation (indirect effect by influencing the secretion of pituitary gland hormones) are performed on adult female rats (body weight of 220-250 g). The substances are subcutaneously administered four times on four consecutive days. The first administration is carried out in the metestrus. One day after the last administration, the autopsy is carried out. The number of oocytes in the Fallopian tube (effect on the ovulation) as well as the uterus weight are determined.

While estradiol produces a dose-dependent ovulation inhibition and an increase in uterus weight with an $ED_{50}$ of 0.004 mg/kg of body weight, the substance according to the invention does not exert any effect on pituitary gland and uterus weight.

Ovary Studies:

The compounds are tested in vivo on hypophysectomized juvenile rats. In a modification of this operative method, a GnRH antagonist (Cetrorelix) is administered to the animals. It is examined whether the substance stimulates follicular proliferation (maturation) in the ovary. The ovary weight is the measurement parameter.

In each case, five animals (body weight 40-50 g) are assigned randomly to the treatment groups. The animals are fed at libitum with a standard diet (altromin) in Makrolon cages in air-conditioned rooms with a lighting program (12 hours of darkness, 12 hours of light) and are given acidified tap water to drink. For the s.c administration, the test substance as well as the control substance (estradiol E2) are dissolved in benzylbenzoate/castor oil (1+4 v/v).

Juvenile female rats are either hypophysectomized on day 0 and subcutaneously treated (administration 1 x daily) from day 1 to day 4 with estradiol, the compound according to the invention, or subcutaneously treated (administration 1 x daily) with a vehicle (castor oil/benzyl benzoate). In the modified version of the method, 0.5 mg/animal/day of Cetrorelix is administered to the animals simultaneously with the compound according to the invention or the vehicle and the control substance estradiol over four days of treatment. In both cases, the animals are sacrificed 24 hours after the last administration, and ovary weight as well as follicle stages are determined.

The compounds according to the invention thus shows a clear dissociation of action in the ovary in comparison to the uterus action and the pituitary gland action and is excellently suited for the treatment of female infertility, because of its follicle-stimulating action.

**Production of the Compounds According to the Invention**

The compounds of general formula I according to the invention are produced as described in the examples. Additional compounds of general formula I can be obtained by an analogous procedure using reagents that are homologous to the reagents that are described in the examples.

Etherification and/or esterification of free hydroxy groups is carried out according to methods that are common to one skilled in the art.

The compounds according to the invention can be present in carbon atoms 16 and 17 as α,β-stereoisomers. In the production of compounds according to the described processes, the compounds in most cases accumulate as mixtures of the corresponding α,β-isomers. The mixtures can be separated by, for example, chromatographic processes.

According to general formula I, possible substituents can already be present in final form or in the form of a

precursor even in the starting product, a substituted estrone already corresponding to the desired end product.

17-Substituents are also introduced according to known processes by nucleophilic addition of the desired substituent or a reactive precursor thereof and are optionally further built up.

The 8β-substituted estratriene-carboxylic acid esters according to the invention are produced from the corresponding hydroxy steroids analogously to processes that are also known (see, e.g., Pharmazeutische Wirkstoffe, Synthesen, Patente, Anwendungen [Pharmaceutical Active Ingredients, Syntheses, Patents, Applications]; A. Kleemann, J. Engel', Georg Thieme Verlag Stuttgart 1978, Arzneimittel, Fortschritte [Pharmaceutical Agents, Improvements] 1972 to 1985; A. Kleemann, E. Lindner, J. Engel (Editors), VCH 1987, pp. 773-814).

The estratriene-sulfamates according to the invention are available in a way that is known in the art from the corresponding hydroxy steroids by esterification with sulfamoyl chlorides in the presence of a base (Z. Chem. 15, 270-272 (1975); Steroids 61, 710-717 (1996)).

Subsequent acylation of the sulfamide group results in the (N-acyl)sulfamates according to the invention, for which pharmacokinetic advantages were already detected in the case of the absence of an 8-substituent (cf. DE 195 40 233 A1).

The regioselective esterification of polyhydroxylated steroids with N-substituted and N-unsubstituted sulfamoyl chlorides is carried out according to partial protection of those hydroxyl groups that are to remain unesterified. Silyl ethers have turned out to be protective groups with selective reactivity that is suitable for this purpose, since these silyl ethers are stable under the conditions of sulfamate formation, and the sulfamate group remains intact when the silyl ethers are cleaved again for regeneration of the residual hydroxyl group(s) still contained in the molecule (Steroids 61, 710-717 (1996)). The production of the sulfamates according to the invention with one or more additional hydroxyl groups in the molecule is also possible in that the starting material is suitable hydroxy-steroid ketones. First, depending on the goal, one or more hydroxyl groups that are present are subjected to sulfamoylation. Then, the sulfamate groups optionally can be converted with a desired acyl chloride in the presence of a base into the (N-acyl)sulfamates in question. The now present oxosulfamates or oxo-(N-acyl)sulfamates are converted by reduction into the corresponding hydroxysulfamates or hydroxy-(N-acyl)sulfamates (Steroids 61, 710-717 (1996)). Sodium borohydride and the borane-dimethyl sulfide complex are suitable as suitable reducing agents.

The introduction of variable substituents in rings D of the estratriene skeleton, particularly the halogen atom (for example a fluorine atom) at C-atom 16, can basically be carried out according to the chemical teaching that is known to one skilled in the art, with which the corresponding estratriene derivatives that are not substituted in 8-position are produced (see, i.a.: Steroide [Steroids], L. F. Fieser, M. Fieser, Verlag Chemie, Weinheim/Bergstr., 1961; Organic Reactions in Steroid Chemistry, J. Fried, J. A. Edwards, Van Nostrand Reinhold Company, New York, Cincinnati, Toronto, London, Melbourne, 1972; Medicinal Chemistry of Steroids, F. J. Zeelen, Elsevier, Amsterdam, Oxford, New York, Tokyo, 1990).

Substituents according to general formula I can also be introduced in the stage of estratrienes that are already substituted in 8-position, according to known methods in the art. This can be useful or necessary especially in the case of multiple substitutions of the desired final compound.

Characteristic, but not limiting synthesis processes, which are useful for providing representative substitution patterns on the estrone skeleton, also in combination with several substituents, are found in, for example: C(1) J. Chem. Soc. (C) 1968, 2915; C(7) Steroids 54, 1989, 71; C(8α) Tetrahedron Letters 1991, 743; C(8β) Tetrahedron Letters 1964, 1763; J. Org. Chem. 1970, 35, 468; C(11) J. Steroid Biochem. 31, 1988, 549; Tetrahedron 33, 1977, 609 and J. Org. Chem. 60, 1995, 5316; C(9) DE-OS 2035879; J. Chem. Soc. Perk. 1 1973, 2095; C(15) J. Chem. Soc. Perk. 1 1996, 1269.); C(13α) Mendeleev Commun. 1994, 187; C(14β) Z. Chem. 23, 1983, 410.

The examples below are used for a more detailed explanation of the invention.

Analogously to the degradation of the 8β-vinyl grouping, other compounds of general formula I can be obtained with use of reagents that are homologous to the reagents that are described in the examples.

Etherification and/or esterification of free hydroxy groups is carried out according to the methods that are common to one skilled in the art.

**Example 1**

**8β-Vinyl-16α-Fluoro-estra-1,3,5(10)-triene-3,17-diol**

3-Methoxy-16α-fluor-8-vinyl-estra-1,3,5(10)-trien-17-one

7,3 g (23,5 mmol) of 3-methoxy-8-vinyl-estra-1,3,5(10),16-trien-17-one in 65 ml THF are added dropwise under argon to 30 ml of a 2M solution of lithium-diisopropylamide (60,0 mmol) in THF / Heptan / Ethylbenzol, cooled down at -78°C, and then 16 ml (115,4 mmol) Triethylamin and 7,6 ml (59,9 mmol) chlortrimethylsilane are added one after the other. The reaction mixture is subsequently warmed up to room temperature in 1,5 h, washed with sodium bicarbonate

solution, and extracted with n-Hexane. The collected organic phases are washed with water, drayed over sodium sulphate and concentrated by evaporation in a vacuum.

The obtained (3-methoxy-8-vinyl-estra-1,3,5(10),16-trien-17-yloxy)-trimethylsilane crude product (12 g of a yellowish liquid residue) is used without any further purification in the next step.

$$R_f = 0{,}54 \text{ (Cyclohexane / Ethyl Acetate = 8 / 2)}$$

6,8 g of the (3-Methoxy-8-vinyl-estra-1,3,5(10),16-tetraen-17-yloxy)-trimethylsilane crude product are dissolved in 50 ml methylenchloride, combined with 5g (15,9mmol) N-fluordibenzensulfonimide and mixed for 16 hours at room temperature in the absence of light. 25 ml 2N hydrochloric acid are added, the organic phase is separated and extracted several times with methylchloride. The collected organic phases are washed with a sodium bicarbonate solution, with water and with a saturated sodium chloride solution one after the other, drayed over magnesium sulphate and concentrated in vacuo. The obtained crude product (8.43 yellowish-brown oil) is purified by chromatography on silica gel (19/1 cyclohexane/ethyl acetate). In this way 1.65g (38%, colourless foam) of 3-methoxy-16α-fluor-8-vinyl-estra-1,3,5(10)-trien-17-one and 2,08g (51% colourless foam) of 3-methoxy-8-vinyl-estra-1,3,5(10)-trien-17-one are obtained.

3-Methoxy-16α-fluor-8-vinyl-estra-1,3,5(10)-trien-17-one 1H-NMR (CDCl3): δ = 0,96 (s, 3H; 18-CH3), 3,76 (s, 3H; OMe), 5,01 (d, 1H; CH=CH2), 5,05 / 5,18 (d, 1H; 16-H), 5,13 (d, 1H; CH=CH2), 5,54 (dd, 1H; CH=CH2), 6,59 (d, 1H; 4-H), 6,69 (dd, 1H; 1-H, 2-H), 7,15 (d, 1H; 1-H, 1-H).

**3-Methoxy-16a-fluor-8-vinyl-estra-1,3,5(10)-trien-17α-ol und 3-methoxy-16α-fluor-8-vinyl-estra-1,3,5(10)-trien-17β-ol**

1,65 g (5,0 mmol) of 3-Methoxy-16α-fluor-8-vinyl-estra-1,3,5(10)-trien-17-one are dissolved in a mixture of 150 ml of THF and 150 ml of Methanol, cooled at 5˚C and combined to 1,9 g (50,2 mmol) of sodiumborohydride. During the warming up to room temperature in 1,5h, the reaction mixture is stirred. 10 ml of acetic acid are then added and the solution finally dried in vacuo. The residue is redissolved in water and extracted several times with ethyl acetate. The organic phases are collected, dried with magnesium sulfate and concentrated in vacuo.

The obtained crude product is separated by chromatography on silica gel (Cyclohexane / Methyl-tert.-Buthylether = 13 /1). According to this procedure 0,67 g (40%) of 3-methoxy-16α-fluor-8-vinyl-estra-1,3,5(10)-trien-17α-ol and 1,00 g (60%) 3-of methoxy-16α-fluor-8-vinyl-estra-1,3,5(10)-trien-17β-ol are obtained in the form of a colorless foam. 3-Methoxy-16α-fluor-8-vinyl-estra-1,3,5(10)-trien-17α-ol 1H-NMR (CDCl3): δ = 0,72 (s, 3H; 18-CH3), 3,72 (d, 1H, 17-H), 3,75 (s, 3H; OMe), 4,90 (d, 1H; CH=CH2), 5,04 (d, 1H; CH=CH2), 5,14/5,28 (dd, 1H; 16-H), 5,51 (dd, 1H; CH=CH2), 6,57 (d, 1H; 4-H), 6,67 (dd, 1H; 1-H, 2-H), 7,16 (d, 1H; 1-H, 1-H).

3-Methoxy-16α-fluor-8-vinyl-estra-1, 3, 5 (10) -trien-17β-ol
1H-NMR (CDCl3): δ = 0,79 (s, 3H; 18-CH3), 3,75 (s, 3H; OMe), 3,81 (d, 1H, 17-H), 4,82 / 4,95 (dd, 1H; 16-H), 4,89 (d, 1H; CH=CH2), 5,06 (d, 1H; CH=CH2), 5,51 (dd, 1H; CH=CH2), 6,57 (d, 1H; 4-H), 6,68 (dd, 1H; 1-H, 2-H), 7,15 (d, 1H; 1-H, 1-H).

**16α-Fluor-8-vinyl-estra-1,3,5(10)-trien-3,17β-diol**

690 mg (1,87 mmol) of tetrabutylammoniumiodide are added to a solution of 70 mg (0,21 mmol) 3-methoxy-16α-fluor-8-vinyl-estra-1,3,5(10)-trien-17β-ol in 9 ml of methylenchloride under argon. The reaction mixture is cooled at -78˚C and then combined with 1,9 ml (1,9 mmol) of a 1M solution ofborotrichloride in methylenchloride. The mixture is stirred for 0,5 h at -78˚C, finally warmed up at room temperature and quenched with water. The organic phase is separated, the water phase is extracted several times with methylenchloride, and the organic phases are collected, washed with a saturated sodium chloride solution, dried with magnesium sulfate and concentrated in vacuo.

The obtained crude product is separated by chromatography on silica gel (Cyclohexane / Ethyl Acetate = 9/1). 26 mg (39%) of 16α-Fluor-8-vinyl-estra-1,3,5(10)-trien-3,17β-diol are obtained according to this procedure.

16α-Fluor-8-vinyl-estra-1,3,5(10)-trien-3,17β-diol 1H-NMR(DMSO[D6]): δ = 0,64 (s, 3H; 18-CH3), 3,49/3,57 (t, 1H, 17-H), 4,70 / 4,84 (dd, 1H; 16-H), 4,85 (d, 1H; CH=CH2), 4,98 (d, 1H; CH=CH2), 5,19 (d, 1H, 17-OH), 5,46 (dd, 1H; CH=CH2), 6,37 (d, 1H; 4-H), 6,48 (dd, 1H; 1-H, 2-H), 6,99 (d, 1H; 1-H, 1-H), 8,93 (s, 1H, 3-OH).

Example 2

**16α-Fluor-8-vinyl-estra-1,3,5(10)-trien-3,17α-diol**

295 mg (0,80 mmol) of tetrabutylammoniumiodide are added to a solution of 30 mg (0,09 mmol) of 3-methoxy-16α-fluor-8-vinyl-estra-1,3,5(10)-trien-17α-ol in 4 ml methylenchloride under argon. The reaction mixture is cooled at -78°C and then combined with 0,8 ml (0,8 mmol) of a 1M solution of borotrichloride in methylenchloride. The mixture is stirred for 0,5 h at -78°C, finally warmed up at room temperature and quenched with water. The organic phase is separated, the water phase is extracted several times with methylenchloride, and the organic phases are collected, washed with a saturated Sodium Chloride solution, dried with magnesium sulfate and concentrated in vacuo.

The obtained crude product is separated by chromatography on silica gel (Cyclohexane / Ethyl Acetate =7/3). According to this procedure 27 mg (94%) 16α-Fluor-8-vinyl-estra-1,3,5(10)-trien-3,17α-diol are obtained.

16α-Fluor-8-vinyl-estra-1,3,5(10)-trien-3,17α-diol 1H-NMR (DMSO[D6]): δ = 0,63 (s, 3H; 18-CH3), 3,51 (t, 1H, 17-H), 4,82 (d, 1H, 17-OH), 4,85 (d, 1H; CH=CH2), 4,97 (d, 1H; CH=CH2), 5,05/5,18 (dd, 1H; 16-H), 5,47 (dd, 1H; CH=CH2), 6,37 (d, 1H; 4-H), 6,48 (dd, 1H; 1-H, 2-H), 6,99 (d, 1H; 1-H, 1-H), 8,97 (s, 1H, 3-OH).

## Claims

**1.** 8β-substituted estra-1,3,5(10)-triene derivatives of general formula I

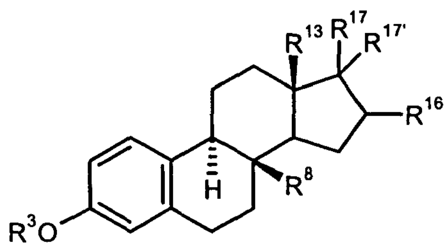

(I)

in which radicals $R^3$, $R^8$, $R^{13}$, $R^{16}$ as well as $R^{17}$ and $R^{17'}$, independently of one another, have the following meaning:

$R^3$ means a hydrogen atom or a group $R^{18}$, in which

$R^{18}$ means a straight-chain or branched-chain, saturated or unsaturated $C_1$-$C_6$-alkyl radical, a trifluoromethyl group,
an aryl, heteroaryl or aralkyl radical, optionally substituted with at least one radical independently chosen from a methyl, ethyl, trifluoromethyl, pentafluoroethyl, trifluoromethylthio, methoxy, ethoxy, nitro, cyano, halo-, hydroxy, amino, mono($C_1$-$C_8$-alkyl) or di($C_1$-$C_8$-alkyl)amino whereby both alkyl groups are identical or different, di(aralkyl)amino whereby both aralkyl groups are identical or different, carboxyl, carboxyalkoxy, $C_1$-$C_{20}$-acyl or $C_1$-$C_{20}$-acyloxy as substituents;
an acyl radical -C(=O)$R^{19}$, in which $R^{19}$ is a straight-chain or branched-chain of a $C_1$-$C_{10}$ -alkyl radical atoms that is saturated or unsaturated in up
to three places and is partially or completely halogenated, or

$R^{18}$ means a group $R^{20}SO_2$, in which

$R^{20}$ is an $R^{21}R^{22}N$ group, whereby $R^{21}$ and $R^{22}$, independently of one another, mean a hydrogen atom, a $C_1$-$C_5$-alkyl radical, a group -C(=O)$R^{23}$, in which R means a unsubstituted or substituted, straight-chain or branched-chain $C_1$-$C_{10}$ -alkyl radical that is saturated or unsaturated in up to three places and is partially or completely halogenated, a cyclopropyl, cyclobutyl, cyclopentyl, cyclohexyl or cycloheptyl group, a $C_4$-$C_{15}$-cycloalkylalkyl radical with 3 to 7 carbon atoms in the cycloalkyl portion and with an alkyl portion of up to 8 carbon atoms or an aryl, heteroaryl or aralkyl radical optionally substituted, with at least one radical independently chosen from a methyl, ethyl, trifluoromethyl, pentafluroethyl, trifluoromethylthio, methoxy, ethoxy, nitro, cyano, halo-, hydroxy, amino, mono($C_1$-$C_8$-alkyl) or di($C_1$-$C_8$-alkyl) amino whereby both alkyl

groups are identical or different, di(aralkyl)amino whereby both aralkyl groups are identical or different, carboxyl, carboxyalkoxy, $C_1$-$C_{20}$-acyl or $C_1$ -$C_{20}$-acyloxy groups as substituents; or,
together with the N atom, a polymethylenimino radical with 4 to 6 C atoms or a morpholino radical,

$R^8$ is a straight-chain or branched-chain alkenyl or alkinyl radical with 2 to 6 carbon atoms, which optionally can be partially or completely fluorinated,
$R^{13}$ is a methyl group or an ethyl group,
$R^{16}$ is a flourine atom,
$R^{17}$ and $R^{17'}$, in each case independently of one another, are a hydrogen atom and a hydroxy group; or a hydrogen atom and a group $R^{18}$O-, $R^{20}SO_2$- or OC(=O)$R^{23}$ with $R^{18}$, $R^{20}$ and $R^{23}$ in each case in the meaning that is indicated under $R^3$

**2.** Compounds of general formula I according to claim 1, in which $R^3$ is a hydrogen atom.

**3.** Compounds of general formula I according to claim 1 or 2, in which $R^8$ is a vinyl, ethinyl or prop-1-inyl group.

**4.** Compounds of general formula I according to any one of the claim 1 to 3 in which $R^{17}$ and $R^{17}$ are a hydrogen atom and a hydroxy group atom.

**5.** Compounds of general formula I according to any one of the claim 1 to 4 in which $R^{16}$ is in α-position.

**6.** Compounds of general formula I according to any one of the claim 1 to 5 in which $R^{16}$ is in β-position.

**7.** Compounds of general formula I to any one of the claim 1 to 6 in which $R^8$ is a vinyl, ethinyl or prop-1-inyl group, $R^{16}$ is a fluorine atom, $R^{17}$ and $R^{17'}$ are a hydrogen atom and a hydroxy group atom.

**8.** Compounds of general formula I to any one of the claim 1 to 8, in which $R^{17}$ and $R^{17'}$ are a hydrogen atom and a group $R^{18}$-O- or $R^{19}SO_2$-O- with $R^{18}$ and $R^{19}$ in each case in the meaning that is indicated under $R^3$.

**9.** Estratrienes of general formula I according to claim 1 or 2, namely:
8β-vinyl- 16α-fluoro-estra-1,3,5(10) -triene-3,17α-diol
8β-vinyl- 16α-fluoro-estra-1,3,5(10)-triene-3,17β-diol
8β-vinyl-16β-fluoro-estra-1,3,5(10)-triene-β,17β-diol

**10.** Pharmaceutical compositions that contain at least a compound according to one of claims 1 to 9 as well as a pharmaceutically compatible vehicle.

**11.** Use of the compounds of general formula I as defined in any one of the claims 1 to 9 for the production of a pharmaceutical agents.

**12.** Use according to claim 11 for treatment of estrogen-deficiency-induced diseases and conditions in women and in men.

**13.** Use according to claim 11 for treatment of peri- and postmenopausal symptoms.

**14.** Use according to claim 11 for the in-vitro treatment of female infertility.

**15.** Use according to claim 11 for the in-vivo treatment of female infertility.

**16.** Use according to claim 11 for the therapy of hormone-deficiency-induced symptoms in ovarian dysfunction that is caused by surgery, medication, etc.

**17.** Use according to claim 11 for hormone replacement therapy (HRT).

**18.** Use according to claim 12 in combination with selective estrogen receptor modulators (SERM), for example raloxifene.

**19.** Use according to claim 11 for prophylaxis and therapy of rheumatoid arthritis multiple sclerosis, and lupus.

**20.** Use according to claim 11 for prophylaxis and therapy of inflammatory intestinal diseases and in particular Crohn's disease

**21.** Use according to claim 11 for prophylaxis and therapy of inflammatory diseases of the skin and in particular psoriasis.

**22.** Use according to claim 11 for prophylaxis and therapy of cardiovascular diseases.

**23.** Use according to claim 11 for prophylaxis and therapy of arteriosclerosis, high blood pressure and hypertensive heart disease.

**24.** Use according to claim 11 for prevention and treatment of prostate hyperplasia.

**25.** Use according to claim 17 in combination with antiestrogens and/or selective estrogen receptor modulators (SERM) for prophylaxis and therapy of prostate hyperplasia.

**26.** Use according to claim 11 for treatment of diseases of the immune system.

**27.** Use of the compounds according to claim 11 for the treatment of endometriosis.

**28.** Use of the compounds according to claim 11 for the treatment of cancer of the colon and small intestine.

**29.** Use of the compounds according to claims 11 for the stimulation of hair growth.

European Patent Office

# EUROPEAN SEARCH REPORT

Application Number
EP 07 07 5600

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br><br><br>A | WO 01/77139 A (SCHERING AG [DE]; PETERS OLAF [DE]; HILLISCH ALEXANDER [DE]; THIEME IN) 18 October 2001 (2001-10-18)<br>* page 1, paragraph 1; claim 20; examples 4,7,8,11-13 *<br>----- | 1-4,6-29<br><br><br>5 | INV.<br>C07J1/00<br>A61K31/565<br>A61P5/30 |
| A | YOO JEONGSOO ET AL: "Synthesis of an estrogen receptor beta-selective radioligand: 5-[F-18]Fluoro-(2R*,3S*)-2,3-bis(4-hydroxy phenyl)pentanenitrile and comparison of in vivo distribution with 16 alpha-[F-18]Fluoro-17 beta-estradiol"<br>JOURNAL OF MEDICINAL CHEMISTRY,<br>vol. 48, no. 20, October 2005 (2005-10), pages 6366-6378, XP002458224<br>ISSN: 0022-2623<br>* page 6367, column 1, paragraph 2 *<br>* page 6369, column 1; table 2 *<br>----- | 1-29 | |
| Y<br><br><br><br><br><br><br><br><br><br>A | VANBROCKLIN HENRY F ET AL: "Fluorine-18 16-beta-( intg luoro)estrogens: Systematic investigation of a new series of fluorine-18-labeled estrogens as potential imaging agents for estrogen-receptor-positive breast tumors"<br>JOURNAL OF MEDICINAL CHEMISTRY,<br>vol. 36, no. 11, 1993, pages 1619-1629, XP002458225<br>ISSN: 0022-2623<br>* page 1621; table II *<br>* page 1622; table V *<br>----- | 1-4,6-29<br><br><br><br><br><br><br><br><br><br>5 | TECHNICAL FIELDS SEARCHED (IPC)<br>C07J<br>A61K<br>A61P |

The present search report has been drawn up for all claims

| Place of search | Date of completion of the search | Examiner |
|---|---|---|
| The Hague | 12 November 2007 | Watchorn, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 07 07 5600

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2007

| Patent document cited in search report | Publication date | Patent family member(s) | Publication date |
|---|---|---|---|
| WO 0177139 A | 18-10-2001 | AT 363487 T | 15-06-2007 |
| | | AT 302790 T | 15-09-2005 |
| | | AU 5834101 A | 23-10-2001 |
| | | AU 7394501 A | 23-10-2001 |
| | | BG 107173 A | 30-05-2003 |
| | | BR 0109983 A | 25-02-2003 |
| | | CA 2406177 A1 | 18-10-2001 |
| | | CN 1433424 A | 30-07-2003 |
| | | CZ 20023382 A3 | 12-02-2003 |
| | | DK 1272504 T3 | 01-10-2007 |
| | | DK 1272505 T3 | 07-11-2005 |
| | | EE 200200589 A | 15-04-2004 |
| | | WO 0177138 A1 | 18-10-2001 |
| | | EP 1272504 A1 | 08-01-2003 |
| | | EP 1272505 A1 | 08-01-2003 |
| | | ES 2245694 T3 | 16-01-2006 |
| | | HR 20020892 A2 | 28-02-2005 |
| | | HU 0300335 A2 | 28-07-2003 |
| | | JP 3828423 B2 | 04-10-2006 |
| | | JP 2003530403 T | 14-10-2003 |
| | | JP 2003534248 T | 18-11-2003 |
| | | MX PA02010066 A | 04-06-2003 |
| | | NO 20024907 A | 05-12-2002 |
| | | NO 20024908 A | 13-11-2002 |
| | | NZ 543724 A | 27-07-2007 |
| | | PL 358667 A1 | 09-08-2004 |
| | | SK 14632002 A3 | 04-03-2003 |
| | | US 2004029847 A1 | 12-02-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0177139 A1
- EP 0346014 A
- EP 9502656 W
- DE 19540233 A1,
- DE OS2035879

### Non-patent literature cited in the description


- **GRADY et al.** *Ann Intern Med,* 1992, vol. 117, 1016-1037
- **HARLAP, S.** *Am J Obstet Gynecol,* 1992, vol. 166, 1986-1992
- **R. F. KAUFFMAN ; H. U. BRYANT.** *DNAP,* 1995, vol. 8 (9), 531-539
- **HUGHESDEN et al.** *Obstet. Gynecol. Survey,* 1982, vol. 37, 59-77
- **ELVIN et al.** *Mol. Cell Endocrinol.,* 1999, vol. 13, 1035-1048
- **MCNATTY et al.** *J. Reprod. Fertil.,* 1999, vol. 54, 3-16
- **WHITE et al.** *J. Clin. Endocrinol. Metab.,* 1996, vol. 81, 3821-3824
- **KUIPER et al.** *Proc. Natl. Acad. Sci.,* 1996, vol. 93, 5925-5930
- **MOSSELMAN ; DIJKEMA.** *Febs Letters,* 1996, vol. 392, 49-53
- **TREMBLAY et al.** *Molecular Endocrinology,* 1997, vol. 11, 353-365
- **KUIPER et al.** *Endocrinology,* 1996, vol. 138, 863-870
- **COUSE et al.** *Endocrinology,* 1997, vol. 138, 4612-4613
- **ONOE, Y. et al.** *Endocrinology,* 1997, vol. 138, 4509-4512
- **REGISTER, T. C. ; ADAMS, M. R.** *J. Steroid Molec Biol,* 1998, vol. 64, 187-191
- **KUIPER, G. J. M. et al.** *Endocrinology,* 1997, vol. 138, 863-870
- **CAMPBELL-THOPSON.** *BBRC,* 1997, vol. 240, 478-483
- **MOSSELMANN, S. et al.** *FEBS Lett.,* 1996, vol. 392, 49-53
- **SHUGRUE et al.** *Steroids,* 1998, vol. 63, 498-504
- **IAFRATI, M. D. et al.** *Nature Medicine,* 1997, vol. 3, 545-548
- **COUSE et al.** *Endocrine Reviews,* 1999, vol. 20, 358-417
- **KREGE, J. H. et al.** *Proc Natl Acad Sci,* 1998, vol. 95, 15677-15682
- **LUBAHN, D. B. et al.** *Proc Natl Acad Sci,* 1993, vol. 90, 11162-11166
- **HESS, R. A. et al.** *Nature,* 1997, vol. 390, 509-512
- **KREGE, J. H.** *Proc Natl Acad Sci,* 1998, vol. 95, 15677-15682
- *J. Steroid Biochem. Molec. Biol,* 1995, vol. 55, 395-403
- *Exp. Opinion Invest. Drugs,* 1998, vol. 7, 575-589
- **HEATHER, H.A.** *Mol Endocrinol.,* 23 March 2006, vol. 21 (1), 1-13
- Ullmans Encyklopädie der technischen Chemie. 1953, vol. 4, 1-39
- **CZETSCH-LINDENWALD.** Hilfsstoffe für Pharmazie und angrenzende Gebiete. *Journal of Pharmaceutical Sciences,* 1963, vol. 52, 918 ff
- *Pharm. Ind.,* 1961, vol. 2, 72
- **DR. H. P. FIEDLER.** Lexikon der Hilfsstoffe für Pharmazie, Kosmetik und angrenzende Gebiete. 1971
- **TESTAS, J. et al.** *Endocrinology,* 1981, vol. 109, 1287-1289
- **STACK ; GORSKI.** *Endocrinology,* 1985, vol. 117, 2024-2032
- **FUHRMANN, U. et al.** *Contraception,* 1995, vol. 51, 45-52
- **A. KLEEMANN ; J. ENGEL.** Pharmazeutische Wirkstoffe, Synthesen, Patente, Anwendungen. Georg Thieme Verlag, 1978
- **ARZNEIMITTEL ; FORTSCHRITTE.** Pharmaceutical Agents, Improvements. VCH, 1972, 773-814
- *Z. Chem.,* 1975, vol. 15, 270-272
- *Steroids,* 1996, vol. 61, 710-717
- **L. F. FIESER ; M. FIESER.** Steroids. Verlag Chemie, 1961
- **J. FRIED ; J. A. EDWARDS.** Organic Reactions in Steroid Chemistry. Van Nostrand Reinhold Company, 1972

- **F. J. ZEELEN.** Medicinal Chemistry of Steroids. Elsevier, 1990
- *J. Chem. Soc.,* 1968, 2915
- *Steroids,* 1989, vol. 54, 71
- *Tetrahedron Letters,* 1991, 743
- *Tetrahedron Letters,* 1964, 1763
- *J. Org. Chem.,* 1970, vol. 35, 468
- *J. Steroid Biochem.,* 1988, vol. 31, 549
- *Tetrahedron,* 1977, vol. 33, 609
- *J. Org. Chem.,* 1995, vol. 60, 5316
- *J. Chem. Soc. Perk. 1,* 1973, 2095
- *J. Chem. Soc. Perk. 1,* 1996, 1269
- *Mendeleev Commun.,* 1994, 187
- *Z. Chem.,* 1983, vol. 23, 410